# EUROPÄISCHE PATENTSCHRIFT

(11) **EP 2 834 208 B1**
(45) Veröffentlichungstag und Bekanntmachung des Hinweises auf die Patenterteilung: **07.03.2018**
(21) Anmeldenummer: 13716203.8
(22) Anmeldetag: 03.04.2013
(51) Int. Cl.: C07C 2/76, H01J 37/32, H05B 6/80, C07C 11/24

(54) **VERFAHREN ZUR PLASMATECHNISCHEN HERSTELLUNG VON ACETYLEN**
METHOD FOR PRODUCTION OF ACETYLENE USING PLASMA TECHNOLOGY
PROCÉDÉ POUR LA FABRICATION D'ACÉTYLÈNE PAR UNE TECHNIQUE DE PLASMA

(30) Priorität: 07.04.2012 DE 102012007230
(43) Veröffentlichungstag der Anmeldung: 11.02.2015
(73) Patentinhaber: Spitzl, Ralf, 53842 Troisdorf (DE)
(72) Erfinder: Spitzl, Ralf, 53842 Troisdorf (DE)
(74) Vertreter: Flaccus, Rolf-Dieter
(86) Internationale Anmeldenummer: PCT/EP2013/000983
(87) Internationale Veröffentlichungsnummer: WO 2013/149723

(56) Entgegenhaltungen:
- EP-A1- 0 601 798
- EP-A2- 0 435 591
- WO-A2-2004/010454
- US-A- 4 975 164
- US-A- 5 277 773
- US-A- 5 695 618
- S SUIB: "A Direct, Continuous, Low-Power Catalytic Conversion of Methane to Higher Hydrocarbons via Microwave Plasmas", JOURNAL OF CATALYSIS, Bd. 139, Nr. 2, 1993, Seiten 383-391, XP055065663, ISSN: 0021-9517, DOI: 10.1006/jcat.1993.1034
- TAMÁS KOVÁCS ET AL: "Methane reformation using plasma: an initial study", JOURNAL OF PHYSICS D. APPLIED PHYSICS, Bd. 39, Nr. 11, 7. Juni 2006 (2006-06-07), Seiten 2391-2400, XP020094506, IOP PUBLISHING, BRISTOL, GB ISSN: 0022-3727, DOI: 10.1088/0022-3727/39/11/013
- HEINTZE M ET AL: "Methane conversion into acetylene in a microwave plasma: Optimization of the operating parameters", JOURNAL OF APPLIED PHYSICS, Bd. 92, Nr. 5, 1. September 2002 (2002-09-01), Seiten 2276-2283, XP012057112, AMERICAN INSTITUTE OF PHYSICS. NEW YORK, US ISSN: 0021-8979, DOI: 10.1063/1.1497457
- CHANGSHENG SHEN ET AL: "Methane coupling in microwave plasma under atmospheric pressure", JOURNAL OF NATURAL GAS CHEMISTRY, Bd. 20, 2011, Seiten 449-456, XP002698576,

## Beschreibung

Die Erfindung befasst sich mit einem Verfahren zur plasmatechnischen Herstellung von Acetylen, wobei es sich um eine Gasphasenherstellung handelt.

Es ist bisher bekannt, Acetylen (Ethin, C₂H₂) durch Verfahren herzustellen, die sich der Lichtbogensynthese bedienen. Dazu wird durch Kohlenstoffelektroden mittels eines Lichtbogens ein heißes Plasma in einer Wasserstoffatmosphäre erzeugt.

Nachteile dieses Verfahrens sind der schlechte Wirkungsgrad von in der Regel unter 10 %, eine geringe Selektivität des Prozesses und die hohen thermischen Verluste.

EP 0 435 591 A2 offenbart ein Verfahren zur Umsetzung von Methan zu Acetylen, Ethylen und Wasserstoff. Bei diesem Verfahren wird Methan bzw. ein Methan-Wasserstoff-Gemisch in eine Reaktionszone eingebracht, die mindestens einen länglichen Plasma-Initiator (z. B. Wolframdraht) enthält, und das Methan und der Plasma-Initiator werden einer Mikrowellenstrahlung ausgesetzt. In den Ausführungsbeispielen betrug die eingesetzte Mikrowellenleistung beispielsweise 3,6 W oder 52 W. Der Acetylenanteil im Produktgas lag beispielsweise bei 33,6 Gew.-%, 12,1 Gew.-% oder 19,6 Gew.-%.

EP 0 601 798 A1 beschreibt ein Verfahren, mittels welchem niedermolekulare Kohlenwasserstoffe (z. B. Methan) unter Einwirkung von Mikrowellenstrahlung zu Acetylen und anderen Produkten umgesetzt werden können. Der niedermolekulare Kohlenwasserstoff wird zusammen mit einem Wasserstoffdonor (z. B. Wasserstoff) und Wasser in eine Reaktionszone eingebracht, die mindestens einen länglichen Plasma-Initiator (z. B. Wolframdraht) enthält. In den Ausführungsbeispielen betrug die eingesetzte Mikrowellenleistung durchschnittlich ca. 10 W. Der Acetylenanteil im Produktgas lag beispielsweise bei 0,89 Gew.-%, 2,17 Gew.-%, 4,71 Gew.-% oder 23,98 Gew.-%.

US 4 975 164 A betrifft ein Verfahren zur Umsetzung von Kohlenwasserstoffen (C₂ oder höher) zu Acetylen, Ethylen und Wasserstoff unter Verwendung von Mikrowellenstrahlung. Hierbei wird der Kohlenwasserstoff unter Beimischung von Wasserstoff in eine Reaktionszone eingebracht, die mindestens einen Plasma-Initiator enthält, und der Kohlenwasserstoff wird zusammen mit dem Plasma-Initiator einer Mikrowellenstrahlung ausgesetzt. In den Ausführungsbeispielen lag die eingesetzte Mikrowellenleistung im Bereich von 10 bis 20 W. Der Acetylenanteil im Produktgas lag beispielsweise bei 17,9 Gew.-%, 10,4 Gew.-% oder 25,8 Gew.-%.

US 5 695 618 A beschreibt eine Methode, mittels welcher Methan nach vorheriger Mikrowellenplasma-Aktivierung an oktaedrische Molekularsiebe gekoppelt werden kann, die als Katalysatoren wirken. Dabei wird Methan zu Produkten umgesetzt, die z. B. Ethylen, Ethan, Acetylen, Propan sowie C₄- und C₅-Kohlenwasserstoffe enthalten. In den Ausführungsbeispielen lag die eingesetzte Mikrowellenleistung z. B. bei 60 W oder 40 W. Der Acetylenanteil im Produktgas lag beispielsweise bei 9,9 % oder 25 Gew.-%.

US 5 277 773 A beschreibt ein Verfahren zur Umsetzung von C₁-Kohlenwasserstoffen (oder höheren Kohlenwasserstoffen) zu ungesättigten Kohlenwasserstoffen und Wasserstoff. Bei diesem Verfahren der Kohlenwasserstoff zusammen mit Wasser und molekularer Wasserstoff in eine Reaktionszone eingebracht, die mindestens einen Plasma-Initiator enthält, und die Reaktionszone wird einer Mikrowellenstrahlung ausgesetzt. In den Ausführungsbeispielen betrug die eingesetzte Mikrowellenleistung beispielsweise 10 W oder 3,6 W. Der Acetylenanteil im Produktgas lag beispielsweise bei 2,17 Gew.-%, 4,71 Gew.-%, 23,98 Gew.-% oder 33,6 Gew.-%.

SUIB S et al. ("A Direct, Continuous, Low-Power Catalytic Conversion of Methane to Higher Hydrocarbons via Microwave Plasmas"; JOURNAL OF CATALYSIS, Bd. 139, Nr. 2, 1993, S. 383-391) beschreiben Mikrowellenplasma-Verfahren zur Umsetzung von Methan zu Ethan, Ethylen und Acetylen. Hierbei wird ein Niedrigtemperatur-Plasma mit geringer Leistung (weniger als 100 W; z. B. 60 W) verwendet. Zur Verminderung des Verkokens werden die Auswahl eines geeigneten Katalysators und hohe Durchflussraten empfohlen.

KOVÄCS T et al. ("Methane reformation using plasma: an initial study"; JOURNAL OF PHYSICY D. APPLIED PHYSICS, Bd. 39, Nr. 11, 7. Juni 2006, S. 2391-2400) berichten über Experimente, in denen Methan-Argon-Gemische bei atmosphärischem Druck in einem mikrowelleninduzierten Plasma behandelt wurden. Als Hauptprodukt der Gasphase wurde Acetylen erhalten (Ausbeute 8 bis 82 %). Allerdings war auch die Rußerzeugung beträchtlich; die Beherrschung des Rußproblems wurde als wesentlich für die Weiterentwicklung des Verfahrens angesehen.

HEINTZE M et al. ("Methane conversion into acetylen in a microwave plasma: Optimization of the operating parameters"; JOURNAL OF APPLIED PHYSICS, Bd. 92, Nr. 5, 1. September 2002, S. 2276-2283) beschreiben Experimente, in welchen Methan - unter Beimischung von Argon - in einem Mikrowellenplasma zu Acetylen umgesetzt wurde. Es wurde beobachtet, dass Rußbildung unterdrückt werden kann, wenn ein gepulstes Plasma verwendet wird. Es wurde der Einfluss verschiedener Prozessparameter auf die Acetylenbildung untersucht, z. B. Druck (15-65 mbar), Durchflussrate (33-190 sccm) und Leistung (16-81 W).

CHANGSHENG S et al. ("Methane coupling in microwave plasma under atmospheric pressure"; JOURNAL OF NATURAL GAS CHEMIS-TRY, Bd. 20, 2011, S. 449-456) berichten über Experimente, in welchen Methan - unter Beimischung von Wasserstoff - in einem Mikrowellenplasma zu Acetylen umgesetzt wurde. Je niedriger das molare CH₄/H₂-Verhältnis war, um so höher war die Acetylen-Ausbeute. Mit steigender Mikrowellenleistung erhöhte sich die Acetylen-Ausbeute. Bei Erhöhung der Durchflussgeschwindigkeit stieg die Acetylen-Ausbeute zunächst an und nahm dann wieder ab. Beispielsweise wurde bei einer Durchflussgeschwindigkeit von 500 ml/min, einem molaren CH₄/H₂-Verhältnis von 1/4 und einer Mikrowellenleistung von 800 W eine Acetylen-Ausbeute von 66.0 % erzielt.

WO 2004/010454 A2 offenbart einen Plasmareaktor zur Durchführung von plasmagestützten Umsetzung von Gasen. Als Beispiel wird die Umsetzung von CO₂ und C₂H₄ zu Acetylen beschrieben, wobei CO und H₂O als Nebenprodukte entstehen. Zusätzlich wurde Argon als Prozessgas verwendet; Wasserstoff wurde bei dieser Reaktion nicht eingesetzt. Die Acetylen-Ausbeute wird mit "0,07" angegeben.

Aufgabe dieser Erfindung war es, diese Nachteile zu überwinden und ein Verfahren zur plasmatechnischen Herstellung von Acetylen zur Verfügung zu stellen, das eine optimierte Herstellung von C₂H₂ ermöglicht.

Diese Aufgabe wird durch ein Verfahren zur plasmatechnischen Herstellung von Acetylen gelöst, dadurch gekennzeichnet, dass Methan mit einer Durchflussrate von 2-4 l/min/kW unter Zuleitung von H₂ mit einer Durchflussrate von 10-40 l/min/kW in ein nicht-thermisches Plasma im Reaktionsraum einer Plasmaquelle geleitet wird, so dass im Reaktionsraum ein Druck von 20-300 mbar herrscht, und wobei das Plasma mittels einer Mikrowellenplasmaquelle mit einer Leistung von 3 kW bis 1 MW erzeugt wird.
Auf diese Weise lässt sich eine Konversion von Methan zu Acetylen von 85-99% erreichen.
Vorteil der Plasmakatalyse im nicht thermischen bzw. nicht Gleichgewichts- Plasma ist der gesteigerte Wirkungsgrad, die hohe Selektivität und die geringen thermischen Verluste.
Der Vorteil der Zuleitung von H₂ ist, dass eine Rußbildung unterdrückt wird.
Der Prozess, insbesondere die Rußbildung und/oder der Wirkungsgrad, kann bevorzugt durch Verfahren der Gruppe OES (optische Emissionsspektroskopie), GC (Gaschromatographie) und MS (Massenspektrometrie) überwacht werden.
Die technisch bevorzugten Mikrowellenfrequenzen sind die industriell genutzten Frequenzen von 440 MHz, 915 MHz und 2,45 GHz. Die Verfahren sind aber nicht auf diese Frequenzen begrenzt. Hochfrequenzanregung (HF, UHF/VHF) ist ebenfalls möglich.

Für die Durchführung des erfindungsgemäßen Verfahrens kann eine Vorrichtung verwendet werden, welche eine innenliegende Plasmakammer besitzt, jedoch sind auch außenliegende Plasmakammern geeignet. Der Plasmareaktor wird dabei von den verwendeten Gasen durchströmt. Die Vorrichtung kann in einem niedrigen Druckbereich von bis zu einigen 10 mbar gezündet werden und kommt daher ohne Plasmainitiator aus.

Ferner kann in der Plasmazone der Vorrichtung ein Flackerschutz, vorzugsweise mindestens ein Prallkörper, insbesondere in Zylinder- oder Kegelform, eingebracht sein oder durch tangentiale Anströmung ein Wirbel/Vortex erzeugt werden. Dies dient der Stabilisierung der Plasmazone und ist von Vorteil, da ein instabiles, flackerndes Plasma den Prozess stört, den Schlupf erhöht und zudem die Beiprodukte erhöhen kann. Durch den/die Prallkörper selbst kann ebenfalls ein Teil der im Patentanspruch erwähnten Gase geführt werden. Da so der Kohlenwasserstoff (= Methan) durch die Plasmazone, die sich über dem Prallkörper befindet, geführt wird, kann eine nahezu vollständige Anregung des Kohlenwasserstoffs (Methan) gewährleistet werden. Diese Gaseinspeisung kann auch über mehrere z.B. konzentrische Zonen des oder der Prallkörper erfolgen.

Die Elemente zur Stabilisierung der Plasmazone sind bevorzugt verstellbar bzw. einstellbar ausgeführt, um damit die Strömung durch den Plasmareaktor bzw. den Plasmabereich auf die entsprechenden Volumenströme und Gasmischungen anzupassen.

Ein Prallkörper selbst besteht typischerweise aus Metall oder Kohlenstoff, insbesondere Graphit.

Der Plasmaraum einer zur Durchführung des erfindungsgemäßen Verfahrens geeigneten Vorrichtung kann als Rohrstück ausgeführt sein. Dies hat den Vorteil, dass die Gase einen freien stirnseitigen Zugang und/oder Abgang zur Plasmakammer haben.

Vorteilhaft ist auch eine seitliche Einkopplung der Mikrowellen in den Plasmaraum (Reaktionsraum). Ebenfalls ist die Einkopplung der Mikrowellen über mehrere Koppelstellen vorteilhaft, da hiermit der Leistungsübertrag pro Koppelstelle reduziert werden kann.

Die vorstehend beschriebenen Vorrichtungen sind nicht Gegenstand der vorliegenden Erfindung.

## Patentansprüche

1. Verfahren zur plasmatechnischen Herstellung von Acetylen, **dadurch gekennzeichnet, dass** Methan mit einer Durchflussrate von 2-4 l/min/kW unter Zuleitung von H₂ mit einer Durchflussrate von 10-40 l/min/kW in ein nicht-thermisches Plasma im Reaktionsraum einer Plasmaquelle geleitet wird, so dass im Reaktionsraum ein Druck von 20-300 mbar herrscht, und wobei das Plasma mittels einer Mikrowellenplasmaquelle mit einer Leistung von 3 kW bis 1 MW erzeugt wird.

## Claims

1. A method for the production of acetylene using plasma technology, **characterized in that** methane is passed at a flow rate of 2-4 l/min/kW into a non-thermal plasma in the reaction chamber of a plasma source while feeding in H₂ at a flow rate of 10-40 l/min/kW, so that in said reaction chamber there is a pressure of 20-300 mbar, said plasma being produced at a power of 3 kW to 1 MW by means of a microwave plasma source.

## Revendications

1. Procédé pour la production d'acétylène par une technique de plasma, **caractérisé en ce que** du méthane est guidé à un débit de 2-4 l/min/kW, avec introduction de H₂ à un débit de 10-40 l/min/kW, dans un plasma non thermique dans l'espace de réaction d'une source de plasma, de manière telle qu'une pression de 20-300 mbars règne dans l'espace de réaction, et le plasma étant généré à l'aide d'une source de plasma à micro-ondes à une puissance de 3 kW jusqu'à 1 MW.
